Europäisches Patentamt

European Patent Office

Office européen des brevets

(11) Publication number: **0 143 748**
A 1

# EUROPEAN PATENT APPLICATION

(21) Application number: 84810565.6

(22) Date of filing: 20.11.84

(51) Int. Cl.⁴: **A 61 N 1/32**, A 61 N 1/34

(30) Priority: 24.11.83 IT 4352083

(43) Date of publication of application: 05.06.85
Bulletin 85/23

(84) Designated Contracting States: **AT BE CH DE FR GB IT
LI LU NL SE**

(71) Applicant: **MASTADO S.A., Via Lavizzari 2A,
CH-6900 Lugano (CH)**

(72) Inventor: **Dominici, Sergio, Via Montevile 16/E,
I-06087 Ponte S. Giovanni (Perugia) (IT)**
Inventor: **Staffolani, Nicola, Via Ominici R. 171,
I-06100 Perugia (IT)**
Inventor: **Maggiore, Enzo, Via S. Bernardino da Siena,
I-06088 S. Maria degli Angeli Perugia (IT)**

(74) Representative: **Baggiolini, Raimondo et al, Racheli &
Fiammenghi Via San Gottardo 15, CH-6900 Lugano (CH)**

(54) Device for prophylaxis and care of tumours, caries and other diseases with notable reduction of pain, by electric and/or magnetic pulses.

(57) As a result of "in vitro" experiments on bacterial cultures and "in vivo" experiments, it was verified that with electrical stimuli with a waveform similar or deriving from that of the physiological signals transmitted by nerve fibers, it is possible to reduce even to elimination of the microbial flora present. These electrical stimuli applied in the oral cavity, reduce even to elimination of germs in the deeper stratum of the dental caries, with a signal of an amplitude less than the person's threshold of sensitivity to the electrical stimulus and do not cause any undesirable side effects; further, they raise the threshold of pain in patients affected with dental neuralgia to almost total disappearance of the neuralgia. Further developments show the possibility to apply said electrical and/or magnetical stimuli or pulses to the prophylaxis and care of tumours, leukemias, sclerosis, diabets and other diseases, and to control the pathological and biological processes on human and living beings.

$$V(t)$$

$$T = \frac{1}{f}$$

0143748

## Device for prophylaxis and care of tumours, caries and other diseases with notable reduction of pain, by electric and/or magnetic pulses.

This invention has as its object a device for the prophilaxis and care of tumours, leukemias, sclerosis, diabetes, dental caries and other diseases without causing undesiderable side effects, raising the threshold of pain, and for control the pathological and biological processes on human and living being, by unicellular or pluricellular species, in animal and vegetable kingdom, as well as for retard the cells ageing in cosmetic filed, characterized by suitable equipment to produce electric and/or magnetic pulses in wave form similar to, or deriving from, the physiological signals transmitted by the nerve fibers.

It was verified, by laboratory experiments, that microorganisms subjected to electrical stimulation with signals generated by the device in question, are neutralized in times of the order of a few minutes depending on the species.

By "in vivo" experiments on teeth affected with penetrating and nonpenetrating caries and subjected to electrical stimulation as above, it was verified that the microbial flora is reduced in times on the order of a few minutes.

It is known that electrostimulation causes analgesia which continues when the stimulation ceases.

In the present state of the art, electric stimulators are produced only for electroanalgesia.

Further, of the stimulators for electroanalgesia produced so far:

a) some, use the extra voltage of a transformer gap, and therefore the signal is of the pulse type, with a high open-circuit peak voltage, with a very steep rise and fall

- 2 -

0143748

front of the signal.

b) other stimulators for electroanalgesia generate pulses of the rectangular type, even if with a lower peak voltage, and obviously they do not work well for the specified aims.

This invention makes it possible, by electric and/or magnetic pulses transmitted to the tissues in question, besides raising the threshold of pain, to destroy microorganisms and to reach the aims as cited above.

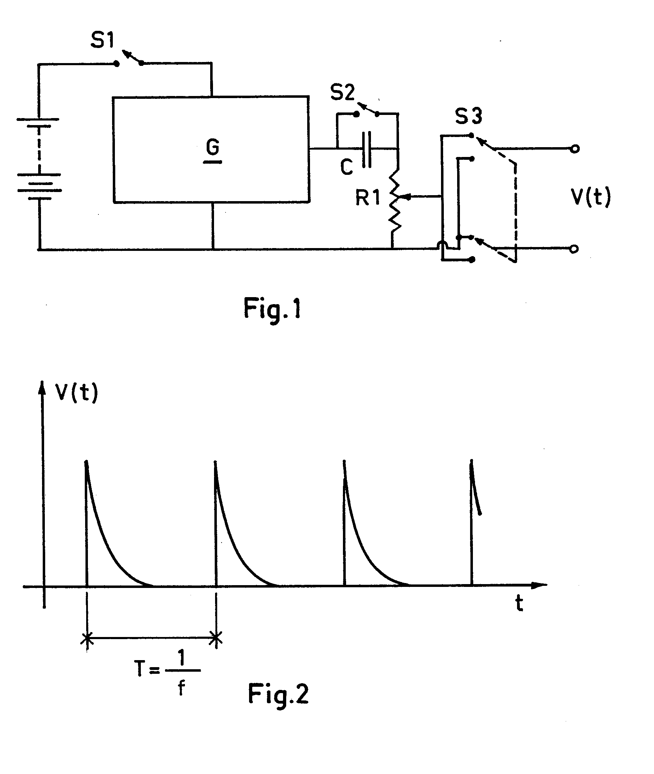

A preferred embodiment of the present invention, relating to the prophilaxis and care of dental caries, will now explained with reference to the enclosed drawing, in which Fig. 1 represents the device and fig. 2 the waves form of electrical pulses.

The device consists of an electric pulse generator G (fig. 1) with a variable frequency from 0,1 to 2500 Hz, supplied by a battery;

- the waveform of the pulses is a curvilinear triangle (see fig. 2) whose rise time is less that a microsecond and fall time can vary between 1/100 (one one hundredth) and 1/3 (one third) of the period lapsing between two contiguous successive pulses;

- the fall front is exponential of the type $e^{-t/\tau}$ where $\tau$ is the time constant of the wave-forming circuit;

- the amplitude of the pulses is varied between 0,1 and 15 volts by potentiometer $R_1$;

- the supplied power which can vary from 1 to 100 microamperes, is varied by $R_1$ so as not to exceed the threshold of individual sensitivity to the electric signal;

- the continuous component of the signal is eliminated by

opening switch $S_2$;

- inversion of the signal is obtained by switch $S_3$.

The electrodes consist of conductors that are nonoxidizably by contact with the tissue, such as carbonated rubber, stainless steel, platinum, titanium, etc. The electric pulses are applied to the oral cavity with suitable electrodes that make it possible to achieve the desired stimulation.

Obviously, there are various solutions in the way of embodying the electrodes and applying them, depending on cases;

1) a first solution is that of using a suitable brush, in whose handle the device in question can be incorporated, having two electrodes, one at the handle, which makes it possible to close the electric circuit by the arm and the other at the bristles or wires which can be made to be conductive.

2) a second solution is to apply two outside electrodes on the cheek in case of neuralgia (optionally reducing the resistance of contact with the skin by using electrolytic pastes).

3) a third solution is to use an electrode with the conductive end (either of carbonated rubber or wire for canal applications and with the electrode in the shape of a horseshoe to press the dental arch, etc.) to be applied to the tooth or teeth in question; while the other electrode can be grasped by the hand or applied to any other part of the body.

According to the present invention, for the prophilaxis and care of tumours, leukemias, sclerosis, diabetes and other diseases, as well as for control the pathological and bio-

- 4 -                                    0143748

logical processes on human and living being, by unicellular or pluricellular species, in animal and vegetable kingdom, and to retard the cells ageing in cosmetic field, the complex waves form of the electric and/or magnetic pulses may satisfy to following requirements:

a) have a rise front very steep (rising time 1 nanosecond) and fall front given from the summation of more exponential terms;

b) frequency comprised from 0,01 Hertz to 250 Kilohertz;

c) frequency variable into a pre-established gap;

d) pulses in form of a sequence of trains, with variable intervals of signals and of not signals;

e) pulses with peak voltages from 0,1 volt to 150 volt and peak currents from 1 microampere to 40 milliamperes.

Electrodes and conductors coming from the device being provided to be applied to the biological systems.

## Claims

1. Device for the prophylaxis and care of tumours, leukemias, sclerosis, diabetes, dental caries and other diseases without causing undesiderable side effects, raising the threshold of pain, and for control the pathological and biological processes on human and living being, by unicellular or pluricellular species, in animal and vegetable kingdom, as well as for retard the cells ageing in cosmetic field, characterized by suitable equipment to produce electric and/or magnetic pulses in wave form similar to, or deriving from, the physiological signals transmitted by the nerve fibers.

2. Device as in claim 1, applied for the prophylaxis and care of dental caries and disinfecting the oral cavity, wherein said equipment produces a variable electrical frequency between 0,1 and 2.500 Hz having the form of a curvilinear triangle whose rise time is less than a microsecond and fall time can vary between 1/100 and 1/3 of the period lapsing between two contiguous successive pulses; the fall front being exponential of the type $e^{-t/\tau}$ where $\tau$ is the time constant of the wave-forming circuit.

3. Device as in claim 2, wherein:

- the amplitude of the pulses being varied between 0,1 and 15 volts by a potentiometer ($R_1$);

- the supplied current, which can vary from 1 to 100 microamperes, is varied by potentiometer ($R_1$) so as not to exceed the threshold of individual sensitivity to the electric signal;

- the continuous component of the signal being eliminated by opening the main switch ($S_2$);

- inversion of the signal being obtained by a switch ($S_3$).

0143748

4. Device as in claims 2 and 3 wherein the electrodes consist of conductors that are not oxidable by contact with the tissues such as carbonated rubber, stainless steel, platinum, titanium and similars, the electric pulses being applied to the oral cavity with electrodes that make it possible to achieve the desired stimulation.

5. Device as in claims 1, 2, 3 and 4 wherein it is incorporated in the handle of a brush and exhibits two electrodes.

6. Device as in claim 5, wherein an electrode is located at the handle and makes it possible to close the electric circuit by the arm and the other is located at the bristles or wires preferably conductive.

7. Device as in claim 5, wherein the two electrodes are external and applied to the cheek in the case of neuralgia, preferably reducing the resistance of contact with the skin by use of electrolytic pastes.

8. Device as in claim 5, wherein an electrode with a conductive end (either of carbonated rubber or wire for canal applications or with an electrode in the shape of a horseshoe to press the dental arch) is to be applied to the tooth or teeth in question, while the other electrode is preferably grasped in the hand or applied to any other part of the body.

9. Device according to claim 1 applied to the prophilaxis and care of tumours, leukemias, sclerosis, diabetes and other diseases, as well as for control the pathological and biological processes on human and living being, by unicellular or pluricellular species, in animal and vegetable kingdom and to retard the cells ageing in cosmetic field, characterized in that the complex waves form of the electric and/or magnetic pulses satisfies to following requirements:

- 7 -

a) they have a rise front very steep (rising time 1 nanosecond) and fall front given from the summation of more exponential terms;

b) frequency from 0,01 Hertz to 250 Kilohertz;

c) frequency variable into a pre-established gap;

d) pulses in form of a sequence of trains, with variable intervals of signals and of not signals;

e) pulses with peak voltages from 0,1 volt to 150 volts and peak currents from 1 microampere to 40 milliamperes, electrodes and conductors coming from the device being provided to be applied to the biological systems.

Fig.1

$$T = \frac{1}{f}$$

Fig.2

**European Patent Office**

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-2 295 585 (R.J.LINDQUIST)<br><br>* Page 1, right-hand column, lines 13-21; page 2, left-hand column, lines 1-52; page 3, right-hand column, lines 24-47 * | 1 | A 61 N 1/32<br>A 61 N 1/34 |
| A | | 3,9 | |
| | --- | | |
| X | FR-A-1 321 127 (S.YAMASHIKI)<br><br>* Page 1, right-hand column, paragraphs 4,5; figure 2 * | 1 | |
| A | | 3,9 | |
| | --- | | |
| A | FR-A-2 296 437 (M.BAULANDE)<br><br>* Page 2, lines 1-20; page 3, line 12 * | 1-3,9 | **TECHNICAL FIELDS SEARCHED (Int. Cl.4)**<br><br>A 61 N<br>A 61 B<br>A 61 C |
| | --- | | |
| A | FR-A-2 153 191 (CHATEAU DE CRESSIA)<br><br>* Page 1,line 22 - page 2, line 12; page 4, lines 7-19 * | 2,3,6,<br>8,9 | |
| | --- | | |
| A | DE-A-2 907 013 (AMERICAN MEDICAL SYSTEMS)<br><br>* Page 5, paragraph 2; page 8, last paragraph *<br>--- | 2,3,7,<br>9 | |
| | -/- | | |

The present search report has been drawn up for all claims

| Place of search | Date of completion of the search | Examiner |
|---|---|---|
| THE HAGUE | 28-02-1985 | SIMON J.J.E. |

European Patent Office

**EUROPEAN SEARCH REPORT**

0143748
Application number

EP 84 81 0565
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | WO-A-79 01 082 (M.NACHMAN)<br><br>* Page 5, paragraph 1; page 7, last paragraph; page 8, paragraphs 1,4; page 9, paragraphs 1,3 *<br><br>--- | 3,4, 6-9 | |
| A | GB-A-2 117 230 (HIROSHI TAKADA)<br><br>* Page 3, lines 44-78 *<br><br>--- | 5,6 | |
| A | FR-A- 699 282 (G.GASENKO)<br><br>* Page 2, lines 7-41 *<br><br>--- | 5,6 | |
| A | DE-A-2 740 188 (W.KRIEGER)<br><br>----- | | |

TECHNICAL FIELDS SEARCHED (Int. Cl.4)

The present search report has been drawn up for all claims

| Place of search | Date of completion of the search | Examiner |
|---|---|---|
| THE HAGUE | 28-02-1985 | SIMON J.J.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82